(19) Europäisches Patentamt
European Patent Office
Office européen des brevets

(11) EP 0 743 063 B1

(12) **FASCICULE DE BREVET EUROPEEN**

(45) Date de publication et mention de la délivrance du brevet:
**13.02.2002 Bulletin 2002/07**

(51) Int Cl.[7]: **A61K 31/16**, C07C 323/60

(21) Numéro de dépôt: **96401061.5**

(22) Date de dépôt: **14.05.1996**

(54) **Utilisation de dérivés d'alpha-aminométhyl-3-4-dichlorobenzyl thioacétamide pour la fabrication d'un médicament inhibant la recapture de la dopamine et nouveaux composés pour cette utilisation**

Verwendung von alpha-Aminomethyl-3,4-dichlorbenzylthioaceamiden zur Herstellung eines dopaminwiederergreifungshemmenden Medikaments sowie Verbindungen zur Verwendung derselben

Use of derivatives of alpha-aminomethyl-3,4-dichlorobenzyl thioacetamide for the preparation of a medicament inhibiting the recapture of dopamine and compounds for this use

(84) Etats contractants désignés:
**AT BE CH DE DK ES FI FR GB GR IE IT LI LU MC NL PT SE**

(30) Priorité: **19.05.1995 FR 9506000**

(43) Date de publication de la demande:
**20.11.1996 Bulletin 1996/47**

(73) Titulaire: **LABORATOIRE L. LAFON**
**94701 Maisons Alfort (FR)**

(72) Inventeur: **Laurent, Philippe**
**69600 Oullins (FR)**

(74) Mandataire: **Le Guen, Gérard et al**
**CABINET LAVOIX 2, place d'Estienne d'Orves**
**75441 Paris Cédex 09 (FR)**

(56) Documents cités:
**EP-A- 0 158 545** **EP-A- 0 406 088**

- **'The Merck Manual of Diagnosis and Therapy, 15th edition', 1987, MERC RESEARCH LABORATORIES * page 1518 ***

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 743 063 B1

## Description

La présente invention concerne l'utilisation en thérapeutique de certains dérivés d'($\alpha$-aminométhyl-3,4-dichlorobenzyl) thioacétamide.

La présente invention concerne plus spécifiquement l'utilisation de ces composés pour inhiber la recapture de la dopamine.

Dans EP-0 158 545 on a déjà décrit une classe de composés de formule

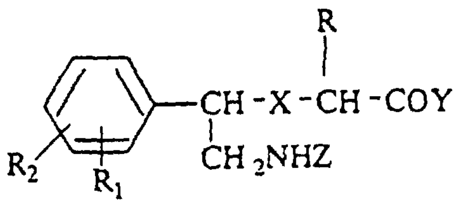

dans laquelle

X est S ou SO,
Y est un groupe alcoxy en $C_1$-$C_4$, un groupe $NH_2$ ou NHOH,
Z est un groupe alkyle en $C_1$-$C_4$,
R est H ou $CH_3$, et
$R_1$ et $R_2$ représentent H, F, Cl ou Br.

Ces composés ont été décrits comme des antidépresseurs.

Dans EP-0 406 088 on a en outre décrit l'[$\alpha$-(tertiobutyl aminométhyl)-3,4-dichlorobenzyl]thioacétamide et ses sels d'addition avec des acides pharmaceutiquement acceptables. Ce composé a été décrit également comme antidépresseur et pour favoriser la prise alimentaire.

La présente invention est basée sur la découverte que :

- l'[$\alpha$-(tertiobutylaminométhyl)-3,4-dichlorobenzyl]thioacétamide et son isomère lévogyre,
- l'[$\alpha$-(tertioamylaminométhyl)-3,4-dichlorobenzyl]thioacétamide et ses isomères,
- l'[$\alpha$-(1-adamantylaminométhyl)-3,4-dichlorobenzyl]thioacétamide et ses isomères,

ainsi que les sels d'addition de ces composés avec des acides pharmaceutiquement acceptables, sont de puissants inhibiteurs de la recapture de la dopamine, et peuvent de ce fait être utilisés en thérapeutique.

La présente invention a en conséquence pour objet l'utilisation d'un composé choisi parmi :

- l'[$\alpha$-(tertiobutylaminométhyl)-3,4-dichlorobenzyl]thioacétamide et son isomère lévogyre,
- l'[$\alpha$-(tertioamylaminométhyl)-3,4-dichlorobenzyl]thioacétamide et ses isomères,
- l'[$\alpha$-(1-adamantylaminométhyl)-3,4-dichlorobenzyl]thioacétamide et ses isomères,

ainsi que les sels d'addition de ces composés avec des acides pharmaceutiquement acceptables, pour la fabrication d'un médicament inhibant la recapture de la dopamine.

L'inhibition de la recapture de dopamine a pour conséquence d'augmenter la concentration dans l'espace synaptique de la dopamine et de la 3-méthoxytyramine (3MT) sans modifier les concentrations d'acide 3,4-dihydroxyphénylacétique (DOPAC) et de l'acide homovanillique (HVA). Cette propriété se traduit par une augmentation du fonctionnement des voies dopaminergiques centrales, objectivée par des modifications comportementales telles que l'apparition de mouvements stéréotypés, l'augmentation de l'activité locomotrice, la réduction de la durée d'immobilité chez des animaux soumis au test du "désespoir comportemental".

En raison de leurs propriétés d'inhibition de la recapture de la dopamine, les composés peuvent être utilisés dans les indications suivantes :

- la stimulation de la cognition,
- la schizophrénie déficitaire,
- la maladie de Parkinson,
- la dépendance pharmacologique vis-à-vis des drogues comme la cocaïne.

Pour les trois premières indications, il existe un déficit de fonctionnement des systèmes dopaminergiques centraux qui peut être corrigé en inhibant la recapture de la dopamine avec comme conséquence une amélioration des transmissions dopaminergiques résultant de l'économie de la dopamine synthétisée et libérée.

Pour la dernière indication, la dépendance pharmacologique vis-à-vis des drogues comme la cocaïne, les substances inhibant la recapture de dopamine se fixent sur le site de transport de la dopamine et entrent donc en compétition sur ce site avec les drogues comme la cocaïne, empêchant ainsi ces drogues d'agir.

Dans ces indications, les composés peuvent être administrés par voie orale à des doses journalières de 0,01 à 10 mg/kg et de préférence de 0,1 à 5 mg/kg.

Plus généralement, les composés peuvent être administrés à l'homme ou aux animaux par voie orale ou parentérale.

Les composés sont généralement administrés sous forme de compositions solides, semi-solides ou liquides.

Comme exemples de compositions, on peut citer les comprimés, les gélules, les suppositoires, les solutions ou suspensions injectables, ainsi que les formes retard et les formes implantées à libération lente. Dans ces compositions, le principe actif est généralement mélangé avec un ou plusieurs excipients pharmaceutiquement acceptables habituels bien connus de l'homme de l'art.

Parmi les composés définis ci-dessus, on préfère l'[α-(tertiobutylaminométhyl)-3,4-dichlorobenzyl]thioacétamide et tout particulièrement l'isomère lévogyre, c'est-à-dire le (-)[α-(tertiobutylaminométhyl)-3,4-dichlorobenzyl]thioacétamide et ses sels d'addition avec des acides pharmaceutiquement acceptables.

La présente invention a en outre pour objet -l'[α-(tertioamylaminométhyl)-3,4-dichlorobenzyl] thioacétamide, ses isomères et les sels d'addition avec des acides pharmaceutiquement acceptables de ces composés,
- l'[α-(1-adamantylaminométhyl)-3,4-dichlorobenzyl] thioacétamide, ses isomères et les sels d'addition avec des acides pharmaceutiquement acceptables de ces composés.

Les isomères de l'[α-(tertiobutylaminométhyl)-3,4-dichlorobenzyl]thioacétamide peuvent être obtenus en séparant les sels diastéréoisomères formés respectivement avec l'acide l(-) malique et l'acide d(+) malique et l'[α-(tertiobutylaminométhyl)-3,4-dichlorobenzyl]thioacétate de méthyle racémique et ammonolyse de chacun des isomères séparés.

Les composés à groupe tertioamyle ou 1-adamantyle peuvent être obtenus selon des procédés analogues à ceux décrits dans EP-A-0 158 545 et EP-A-0 406 088.

Les exemples suivants illustrent la préparation des composés nouveaux.

## EXEMPLE 1

### Préparation du chlorhydrate du l (-) [α-(tertiobutylaminométhyl)-3,4-dichlorobenzyl]thioacétamide (CRL 41 789)

**(1)** Préparation du l(-)malate du (-)[α-(tertiobutylaminométhyl)-3,4-dichlorobenzyl]thioacétate de méthyle

On mélange à 20°C une solution de 42,4g (0,121 mole) de l'[α-(tertiobutylaminométhyl)-3,4-dichlorobenzyl] thioacétate de méthyle racémique (cf. EP-A-0 406 088) dans 100 ml d'acétate d'éthyle avec une solution de 8,2 g (0,061 mole) d'acide l(-)malique dans 200 ml d'acétate d'éthyle, refroidit, essore et recristallise trois fois dans 250 ml d'acétate d'éthyle chaque fois, jusqu'à obtention d'un précipité présentant un point de fusion de 113-114° C.

On obtient le l(-)malate du l(-)amino ester avec un rendement de 35 %.

**(2)** Préparation du chlorhydrate du l(-)[α-(tertiobutylaminométhyl)-3,4-dichlorobenzyl]thioacétamide (CRL 41 789)

On traite 18 g (0,043 mole) du l(-) malate précédent en solution dans 200 ml de méthanol avec 100 ml d'ammoniaque à 28 %; on agite 5 heures, laisse une nuit en contact, évapore le méthanol sous vide, reprend à l'eau, extrait au $CH_2Cl_2$, lave à l'eau, sèche, évapore, reprend le résidu huileux avec 50 ml d'acétone, acidifie avec 10 ml d'une solution d'acide chlorhydrique 5N dans l'isopropanol, essore, sèche et obtient le composé avec un rendement de 24 %.

C'est une poudre crème; il est soluble dans l'eau, l'éthanol; insoluble dans l'éther, l'acétate d'éthyle.

Il fond à 170° C et présente une rotation spécifique $\alpha$ = - 95° ±5° (2 % $CH_3OH$).

**EXEMPLE 2**

**Préparation du chlorhydrate du d (+)[α(tertiobutylaminométhyl) 3,4 dichiorobenzyl]thioacétamide (CRL 41 790)**

**(1)** Préparation du d(+) malate du d(+)[α(tertiobutylaminométhyl) 3,4 dichlorobenzyl]thioacétate de méthyle

On mélange à 20° C une solution de 29 g (0,083 mole) de l'[a(tertiobutylaminométhyl)3,4-dichlorobenzyl] thioacétate de méthyle (mélange racémique récupéré après séparation du CRL 41 789, constitué en majorité de l'isomère dextrogyre) dans 50 ml d'acétate d'éthyle avec une solution de 5,6 g (0,042 mole) d'acide d (+) malique dans 250 ml d'acétate d'éthyle, refroidit, essore et recristallise deux fois dans 200 ml d'acétate d'éthyle, jusqu'à obtention d'un précipité présentant un point de fusion de 114°C.
On obtient le d (+) malate du d (+) amino-ester avec un rendement de 50 %.

**(2)** Préparation du chlorhydrate du d (+)[α(tertiobutylaminométhyl) 3,4-dichlorobenzyl]thioacétamide (CRL 41 790)

On traite 18 g (0,043 mole) du d (+) malate précédent en solution dans 200 ml de méthanol avec 100 ml d'ammoniaque à 28 %; on agite deux heures et laisse en contact 24 heures à l'ambiante.
On évapore le méthanol sous vide, reprend à l'eau, extrait au $CH_2Cl_2$, lave à l'eau, sèche, évapore, reprend le résidu avec 50 ml d'acétone, acidifie avec 10 ml d'une solution d'acide chlorhydrique 5N dans l'isopropanol et essore.
On obtient le composé avec un rendement global de 22 %. Il se présente sous la forme d'une poudre rosée. Il est soluble dans l'eau, l'éthanol, le méthanol, insoluble dans l'éther, l'acétate d'éthyle. Il fond à 170° C et présente une rotation spécifique $\alpha = + 91° \pm 5°$ (2 % $CH_3OH$).

**EXEMPLE 3**

**Préparation du chlorhydrate de l'[α(tertioamylamino méthyl) 3,4 dichlorobenzyl] thioacétamide (CRL 42 059)**

**(1)** Préparation du chlorhydrate du 1-(3,4-dichlorophényl)-2-(tertio amylamino) éthanol

On ajoute à froid 27 g (0,1 mole) de 1-(3,4 dichloro phényl)-2 bromo éthanol dans une solution de 37,5 g (50 ml) (0,43 mole) de 2-méthyl 2-butylamine, 100 ml d'éthanol et 0,5 g de KI. Après 48 heures en contact à 20° C et 3 heures à reflux, on filtre, évapore le filtrat sous vide, reprend le résidu à l'éther, lave à l'eau. On ajoute 200 ml d'HCl N, essore, lave avec 20 ml d'eau froide et à l'éther. On obtient le chlorhydrate (F = 184-185° C) avec un rendement de 78 %.

**(2)** Préparation du chlorhydrate du 1-(3,4-dichlorophényl)-2-(tertioamylamino)-1-chloroéthane

Dans une suspension de 23,4 g (0,075 mole) du chlorhydrate de l'alcool précédent dans 75 ml de $CH_2Cl_2$, on ajoute une solution de 15 ml de $SOCl_2$ dans 40 ml de $CH_2Cl_2$. On chauffe 5 heures à reflux, laisse reposer une nuit, essore, lave avec deux fois 20 ml de $CH_2Cl_2$. On obtient le chlorhydrate (F=200-202°c) avec un rendement de 72%.

**(3)** Préparation du [α-(tertioamylaminométhyl)-3,4 dichlorobenzyl]thioacétate de méthyle

On prépare une solution de méthylate de sodium en dissolvant à froid 2,3 g (0,1 Atg) de sodium dans 120 ml de méthanol.
On ajoute à froid, en agitant, 5 ml (0,05 mole) de thioglycolate de méthyle et 16,55 g (0,05 mole) du chlorhydrate précédent. On agite une heure à l'ambiante et 5 heures au reflux. Après une nuit au repos, on filtre le NaCl, évapore le méthanol sous vide, reprend à l'éther, lave à l'eau, extrait avec HCl N, précipite à froid avec NaOH 3N, extrait à l'éther, lave à l'eau, sèche et évapore.
On obtient l'ester huileux avec un rendement de 90 %.

**(4)** Préparation du chlorhydrate de l'[α(tertioamylamino méthyl) 3,4 dichlorobenzyl] thioacétamide (CRL 42 059)

16 g (0,044 mole) de l'aminoester précédent en solution dans 120 ml de méthanol sont traités avec 40 ml de $NH_4OH$ à 28 %. Après 48 heures en contact à 20° C, on chasse le méthanol sous vide, reprend le résidu à l'eau, extrait à l'éther, extrait celui-ci avec HCl N, précipite avec NaOH concentrée, extrait à l'éther, lave à l'eau sèche, évapore et cristallise la base (F = 84° C) dans l'éther isopropylique.
Cette base en solution dans 100 ml d'acétate d'éthyle est acidifiée avec une solution d'HCl dans l'isopropanol.

On essore, recristallise dans éthanol-acétate d'éthyle et obtient le composé avec un rendement de 60 %.
C'est une poudre blanche, il est soluble dans l'eau et les alcools; insoluble dans l'éther, l'acétate d'éthyle. Il fond à 155-156° C.

## EXEMPLE 4

**Préparation de l'hemifumarate de l'[α(1-adamantylaminométhyl)-3,4-dichlorobenzyl]-thioacétamide (CRL 42 060)**

**(1)** Préparation du chlorhydrate du 1-(3,4-dichlorophényl)-2-(1-adamantylamino) éthanol

Dans une solution de 30 g (0,2 mole) de 1-adamantamine, 0,1 g de KI et 100 ml d'éthanol, on ajoute 27 g (0,1 mole) de 1-(3,4-dichloro phényl)-2-bromoéthanol; on agite 48 heures à 20° C et 6 heures au reflux.
On évapore le méthanol sous vide, reprend le résidu avec 200 ml d'éther et 30 ml HCl 3N; essore, lave avec deux fois 20 ml d'eau froide et à l'éther. On obtient le chlorhydrate (F = 258-260° C) avec un rendement de 70 %.

**(2)** Préparation du chlorhydrate du 1-(3,4-dichlorophényl)-2-(1-adamantylamino)-1-chloroéthane

Dans une suspension de 25 g (0,066 mole) du chlorhydrate de l'aminoalcool précédent dans 60 ml de $CH_2Cl_2$, on ajoute 15 ml de $SOCl_2$ dans 40 ml de $CH_2Cl_2$.
Après 4 heures au reflux et une nuit au repos, on essore, lave au $CH_2Cl_2$ et à l'éther. On obtient le composé chloré avec un rendement de 94 %. Il sublime sans fondre à partir de 250° C.

**(3)** Préparation du chlorhydrate de l'[α(1-adamantylaminométhyl)-3,4-dichlorobenzyl]-thioacétate de méthyle

On prépare une solution de méthylate de sodium avec 2,76 g (0,12 Atg) de sodium et 150 ml de méthanol. On ajoute à froid 6 ml (0,06 mole) de thioglycolate de méthyle et 23,7 g (0,06 mole) du composé chloré précédent. On agite une heure à l'ambiante et 5 heures au reflux.
Après une nuit en contact, on filtre le NaCl, évapore le méthanol sous vide, reprend à l'eau, extrait à l'éther, lave à l'eau. On ajoute 30 ml d'HCl 3N, agite, essore, lave avec deux fois 20 ml $H_2O$ froide et à l'éther.
Le précipité est mis en suspension dans 500 ml $H_2O$, neutralisé avec $Na_2CO_3$ et extrait au $CH_2Cl_2$. On lave, sèche, évapore, reprend à l'acétate d'éthyle et acidifie avec de l'isopropanol chlorhydrique 5N. On essore, lave à l'acétate d'éthyle et obtient le chlorhydrate de l'amino ester (F = 153-154° C) avec un rendement de 65 %.

**(4)** Préparation de l'hemifumarate de l'[α(1-adamantylaminométhyl)-3,4-dichlorobenzyl]-thioacétamide

On sature d'ammoniac une solution froide de 15,5 g (0,033 mole) de chlorhydrate de l'amino ester précédent dans 150 ml de méthanol. On ajoute 10 ml de $NH_4OH$ et encore 10 ml de $NH_4OH$ 24 heures après.
On laisse 48 heures en contact en suivant la formation de l'amide en C.C.M. On évapore le méthanol sous vide, reprend à l'eau, extrait à l'éther, lave à l'eau, sèche et évapore.
On reprend le résidu huileux avec 30 ml d'éthanol, ajoute 2 g d'acide fumarique, essore, lave avec 5 ml d'éthanol froid et 20 ml AcOEt. On recristallise dans l'éthanol et obtient le composé avec un rendement de 77 %.
Il se présente sous la forme d'une poudre blanche.
Il est soluble dans le méthanol, le DMSO, insoluble dans l'eau, l'éther, l'acétate d'éthyle. Il fond à 188° C.
On donnera ci-après des résultats d'essais pharmacologiques mettant en évidence notamment les effets au site de recapture de la dopamine par les composés utilisés dans l'invention, en donnant à titre de comparaison les résultats obtenus avec l'isomère dextrogyre de l'[α-(tertiobutylaminométhyl)-3,4-dichlorobenzyl]thioacétamide.

**Inhibition de la liaison au site de recapture de la dopamine in vitro dans le striatum de rat**

Les rats (mâles, CD Sprague Dawley, 200-250 g) sont sacrifiés par décapitation. Les striata sont immédiatement prélevés, puis conservés à -80° C jusqu'à utilisation. Le jour de la manipulation, les striata sont décongelés, recouverts de tampon, puis mis en suspension. Cette suspension est ensuite versée dans des tubes à centrifuger, complétée jusqu'à 10 ml avec du tampon pH 7,7 (Tris (HCl) 50 mM, NaCl 120 mM, KCl 5 mM), puis centrifugée à 20.000 tours/minute pendant 15 minutes. Le culot est remis en suspension dans 10 ml de tampon puis est soumis à une deuxième centrifugation (20.000 tours/minute pendant 15 minutes). La procédure précédente est répétée, afin que l'homogénat subisse une troisième centrifugation identique aux deux précédentes. Le culot ainsi obtenu est mis en suspension dans quelques ml de tampon, puis homogénéisée. La suspension membranaire est alors diluée dans

un volume nécessaire. Des concentrations croissantes des composés testés en solution dans le tampon sont distribuées. Des aliquots de la suspension membranaire sont ensuite ajoutés. Le ligand radioactif ([$^3$H]-mazindol) utilisé comme marqueur du type de site étudié est alors distribué manuellement dans les tubes qui sont ensuite mis à incuber (volume final 1 ml).

L'arrêt de la réaction se fait par filtration sur un système HARVESTER (bande filtre GF/B WHATMAN). La bande filtre est ensuite lavée trois fois par 5 ml de tampon avant d'être placée dans un système automatique de découpe.

Les filtres coupés tombent dans des fioles de comptage et 4 ml de liquide scintillant sont distribués automatiquement par le système. Chaque échantillon est soumis au comptage de la radioactivité à l'aide d'un compteur à scintillation liquide.

Trois séries d'essais (essai 1, essai 2 et essai 3) sont réalisées avec chaque composé pour la détermination de la $CI_{50}$, chaque détermination étant réalisée sur deux échantillons différents.

La liaison spécifique est définie comme la différence entre la liaison totale et la liaison non spécifique (déplacée par un excès de ligand non radioactif). Les valeurs obtenues en coups par minute (c.p.m.) sont ensuite transformées en désintégrations par minute (d.p.m.) en fonction du rendement du compteur.

La $CI_{50}$ est définie comme la concentration de la substance étudiée, nécessaire pour déplacer 50 % du marqueur radioactif lié spécifiquement.

Les données expérimentales issues du compteur à scintillation liquide (KONTRON) sont stockées sur disquette, gérées par un programme (CONVERT), puis analysées au moyen du logiciel LIGAND* qui calcule la concentration inhibitrice 50 % ($CI_{50}$) et la constante d'inhibition Ki

$$Ki = \frac{CI_{50}}{1+[L]/Kd}$$

où [L] représente la concentration du ligand radioactif et Kd la constante de dissociation de ce même ligand.
(Les valeurs de la $CI_{50}$ et du Ki sont inversement proportionnelles à l'affinité du produit étudié pour le site de liaison).

On donnera dans le tableau I les valeurs moyennes de $CI_{50}$ et Ki obtenus.

On donnera également dans ce tableau les valeurs de $DL_0$ et $DL_{100}$ chez la souris par voie i.p.

TABLEAU I

| Composé | Code | Toxicité mg/kg | | Inhibition de la liaison au site de recapture de la dopamine | |
|---|---|---|---|---|---|
| | | $DL_0$ | $DL_{100}$ | $CI_{50}$ | Ki |
| R = tertiobutyle racémate chlorhydrate | CRL 41 414 | 128 | 256 | $3{,}1.10^{-8}$ | $6{,}3.10^{-8}$ |
| R = tertiobutyle isomère lévogyre chlorhydrate | CRL 41 789 | 64 | 256 | $4{,}4.10^{-8}$ | $2{,}4.10^{-8}$ |
| R = tertiobutyle isomère dextrogyre chlorhydrate | CRL 41 790 | 128 | 512 | $1{,}1.10^{-6}$ | $6{,}2.10^{-7}$ |
| R = tertioamyle racémate chlorhydrate | CRL 42 059 | 30 | 256 | $7{,}4.10^{-8}$ | $4{,}6.10^{-8}$ |
| R = 1-adamantyle racémate chlorhydrate | CRL 42 060 | 128 | 1024 | $4{,}0.10^{-8}$ | $4{,}4.10^{-8}$ |

**Effet sur les taux extracellulaires des neuromédiateurs et des métabolites dans le striatum de rat anesthésié (microdialyse intracérabrale)**

Cette détermination est obtenue par chromatographie liquide à haute performance couplée à une détection électrochimique.

- CLHP : cartouche Sphérisorb, ODS-2 (5 µm), L = 125 M, O = 4mm, MERCK. Phase mobile : phosphate de potassium 0,1 M, acide citrique 0,1 M, acide octane sulfonique 50 mg/l, EDTA 0,1 mM, méthanol 4 %, pH 4,80, débit de la pompe 0,9 ml/min.
- détection éléctrochimique ÷ détecteur BAS, LC-4C, + 750 mV
- analyseur : Millenium, WATERS

Des rats (mâles, SPRAGUE-DAWLEY, CD, Charles RIVER, 280-300 g) anesthésiés à l'hydrate de chloral (400 mg/kg, i.p.) sont placés dans un appareil stéréotaxique. La sonde à microdialyse (CMA/1l L = 3 mm, O = 0,24 mm, CARNEGIE) est implantée suivant les coordonnées de l'atlas de Paxinos et Watson dans la partie antérieure du striatum gauche : I = 10, L = 2,4, H = -6,1.
Du liquide de Ringer ($CaCl_2$ = 2,4 mM, KCl = 4 mM, NaCl = 147 mM) est perfusé à 2 µl/min dans la sonde. Les dialysats recueillis toutes les 20 minutes sont réfrigérés (à environ 6° C) puis analysés.
Après une heure de recueil, des lots de 6 rats reçoivent, par voie intrapéritonéale, sous un volume de 5 ml/kg :

- soit une injection de soluté isotonique de chlorure de sodium,
- soit le composé testé en solution dans l'eau distillée aux doses de 16, 32 ou 64 mg/kg.

Dans le dialysat, les concentrations en dopamine (DA), acide 3,4-dihydroxyphénylacétique (DOPAC), acide homovanillique (HVA), 3-méthoxytyramine (3MT), sont mesurées par détection électrochimique, quantifiées par rapport à une gamme étalon et sont corrigées en fonction du rendement de la sonde effectué in vitro avant chaque essai.
Seuls sont pris en compte les essais dont les sondes présentent un rendement supérieur à 5 %.
On observe avec le composé CRL 41 789 une augmentation significative des concentrations de dopamine et de 3MT, sans modification des concentrations de DOPAC et de HVA, qui confirme les résultats obtenus dans le test de l'inhibition de la dopamine. Il est à noter qu'en revanche l'isomère dextrogyre (CRL 41 790) ne provoque aucune modification des concentrations de dopamine et de 3 MT.

**Induction de mouvements stéréotypés chez le rat**

Des lots de 6 rats (mâles, CD, Sprague-Dawley, Charles River, 160-200 g) reçoivent par voie intrapéritonéale, le CRL 41 789 ou le CRL 41 790 dans de l'eau distillée (5 ml/kg) et sont immédiatement placés dans des cages en Plexiglas (20 x 10 x 10 cm). Les mouvements stéréotypés sont cotés de 0 à 3, toutes les 10 minutes jusqu'à leur disparition.
On observe avec le composé CRL 41 789, aux doses de 16 et 32 mg/kg, l'apparition de mouvements stéréotypés intenses et durables. Par contre, il est à noter que le composé CRL 41 790 ne provoque l'apparition d'aucun mouvement stéréotypé même à la forte dose de 128 mg/kg.

**Effets sur l'activité locomotrice de la souris**

Une demi-heure après avoir reçu par voie intrapéritonéale le CRL 41 789 ou le CRL 41 790 dans de l'eau distillée, des lots de 6 à 24 souris (mâles, NMRI, C.E.R. Janvier, 18-22 g) sont placés individuellement dans des actimètres à faisceaux croisés où leur activité locomotrice (nombre de rayons traversés) est enregistrée pendant 30 minutes.
On observe avec le composé CRL 41 789, aux doses de 2, 8 et 32 mg/kg, une augmentation nette et statistiquement significative, croissant avec la dose, de l'activité locomotrice de la souris. Par contre, il est à noter que le composé CRL 41 790 n'entraîne aucune augmentation de l'activité locomotrice de la souris, même à la forte dose de 64 mg/kg.

**Effets sur le test du "désespoir comportemental" chez la souris**

Une demi-heure après avoir reçu par voie intrapéritonéale le CRL 41 789 ou le CRL 41 790 dans de l'eau distillée, des lots de 6 ou 12 souris (mâles, CD1, Charles River, 18-22 g) sont placés individuellement dans un récipient, haut, en verre transparent, rempli d'eau sur une hauteur de 6 cm. On note la durée totale d'immobilité entre le début de la 3ème minute et la fin de la 6ème minute suivant l'immersion de l'animal.
On observe avec le composé CRL 41 789, aux doses de 0,5, 2,8 et 32 mg/kg, une diminution croissant avec la dose, de la durée d'immobilité. Cet effet est net et statistiquement significatif aux doses de 8 et 32 mg/kg. Par contre, il est à noter que le composé CRL 41 790 n'entraîne aucune diminution de la durée d'immobilité de la souris, même à la forte dose de 64 mg/kg.

## Revendications

1. Utilisation d'un composé choisi parmi :

   - l'[α- (tertiobutylaminométhyl) -3,4-dichlorobenzyl]thioacétamide et son isomère lévogyre,
   - l' [α-(tertioamylaminométhyl)-3,4-dichlorobenzyl]thioacétamide et ses isomères,
   - l'[α-(1-adamantylaminométhyl) -3,4-dichlorobenzyl]thioacétamide et ses isomères,

   ainsi que les sels d'addition de ces composés avec des acides pharmaceutiquement acceptables, pour la fabrication d'un médicament inhibant la recapture de la dopamine, destiné à la stimulation de la cognition, au traitement de la schizophrénie déficitaire, de la maladie de Parkinson et de la dépendance pharmacologique vis-à-vis des drogues.

2. Utilisation selon la revendication 1 dans laquelle on utilise un composé choisi parmi l'[α-(tertiobutylaminométhyl)-3,4-dichlorobenzyl]thioacétamide et son isomère lévogyre.

3. Utilisation selon la revendication 2 dans laquelle le composé est l'isomère lévogyre.

4. [α- (tertioamylaminométhyl)-3,4-dichlorobenzyl]thioacétamide, ses isomères et les sels d'addition avec des acides pharmaceutiquement acceptables de ces composés.

5. [α- (1-adamantylaminométhyl-3,4-dichlorobenzyl]thioacétamide, ses isomères et les sels d'addition avec des acides pharmaceutiquement acceptables de ces composés.

## Claims

1. Use of a compound selected from:

   - [α-(tert-butylaminomethyl)-3,4-dichlorobenzyl]-thioacetamide and its laevorotatory isomer,
   - [α-(tert-amylaminomethyl)-3,4-dichlorobenzyl]-thioacetamide and its isomers,
   - [α-(1-adamantylaminomethyl)-3,4-dichlorobenzyl]-thioacetamide and its isomers,

   as well as the addition salts of these compounds with pharmaceutically acceptable acids, for the manufacture of a medicine inhibiting dopamine re-uptake, intended for the stimulation of cognition, for the treatment of deficit schizophrenia, of Parkinson's disease and of pharmacological drug-dependency.

2. Use according to claim 1, wherein a compound is used selected from [α-(tert-butylaminomethyl.)-3,4-dichlorobenzyl]thioacetamide and its laevo-rotatory isomer.

3. Use according to claim 2, wherein the compound is the laevorotatory isomer.

4. [α-(tert-amylaminomethyl)-3,4-dichlorobenzyl]-thioacetamide, its isomers and the addition salts with pharmaceutically acceptable acids of these compounds.

5. [α-(1-adamantylaminomethyl)-3,4-dichlorobenzyl]-thioacetamide, its isomers and the addition salts with pharmaceutically acceptable acids of these compounds.

## Patentansprüche

1. Verwendung einer Verbindung, die aus

   - [α-(*tert.*-Butylaminomethyl)-3,4-dichlorbenzyl]thioacetamid und seinem linksdrehenden Isomeren

   - [α-(*tert.*-Amylaminomethyl)-3,4-dichlorbenzyl]thioacetamid und seinen Isomeren und

   - [α-(1-Adamantylaminomethyl)-3,4-dichlorbenzyl]thioacetamid und seinen Isomeren

ausgewählt ist, sowie der Additionssalze dieser Verbindungen mit pharmazeutisch verträglichen Säuren zur Herstellung eines die Wiederaufnahme des Dopamins hemmenden Arzneimittels, das zur Stimulation der Kognition und zur Behandlung der Mangel-Schizophrenie, der Parkinson-Krankheit und der pharmakologischen Drogenabhängigkeit vorgesehen ist.

**2.** Verwendung nach Anspruch 1, bei welcher eine Verbindung verwendet wird, die aus [α-(*tert*.-Butylaminomethyl)-3,4-dichlorbenzyl]thioacetamid und seinem linksdrehenden Isomeren ausgewählt ist.

**3.** Verwendung nach Anspruch 2, bei welcher die Verbindung das linksdrehende Isomere ist.

**4.** [α-(*tert*.-Amylaminomethyl)-3,4-dichlorbenzyl]thioacetamid, seine Isomeren und die Additionssalze dieser Verbindungen mit pharmazeutisch verträglichen Säuren.

**5.** [α-(1-Adamantylaminomethyl)-3,4-dichlorbenzyl]thioacetamid, seine Isomeren und die Additionssalze dieser Verbindungen mit pharmazeutisch verträglichen Säuren.